# EUROPEAN PATENT SPECIFICATION

(11) **EP 2 671 603 B1**
(45) Date of publication and mention of the grant of the patent: **10.06.2020**
(21) Application number: 12742106.3
(22) Date of filing: 31.01.2012
(51) Int. Cl.: G16H 40/63, G16H 20/17, A61M 5/145, A61M 5/168, A61M 5/00

(54) **DRUG SOLUTION INJECTION DEVICE**
VORRICHTUNG ZUR INJEKTION VON ARZNEIMITTELLÖSUNGEN
DISPOSITIF D'INJECTION DE SOLUTION DE MÉDICAMENT

(30) Priority: 01.02.2011 JP 2011020009; 27.10.2011 JP 2011236546
(43) Date of publication of application: 11.12.2013
(73) Proprietor: Nemoto Kyorindo Co., Ltd., Tokyo 113-0033 (JP)
(72) Inventor: NEMOTO, Shigeru, Tokyo 113-0033 (JP)
(74) Representative: Plasseraud IP
(86) International application number: PCT/JP2012/052179
(87) International publication number: WO 2012/105577

(56) References cited:
- JP-A- 2003 290 343
- JP-A- 2004 248 734
- JP-A- 2009 285 497
- US-A1- 2004 162 484
- US-A1- 2007 238 989

## Description

### TECHNICAL FIELD

The present invention relates to a fluid injection apparatus (chemical liquid injector) for injecting a contrast medium and physiological saline into a patient. The present invention relates in particular to a fluid injection apparatus in which an injection condition can be set easily in consideration of a weight of a patient, an injection time, and a dose per weight, and the set contents can be reviewed easily.

### BACKGROUND ART

Currently employed medical imaging diagnostic apparatuses include CT (Computed Tomography) scanners, MRI (Magnetic Resonance Imaging) apparatuses, PET (Positron Emission Tomography) apparatuses and the like. In using the abovementioned imaging apparatuses, a contrast medium or physiological saline (hereinafter also referred to simply as a "fluid") is often injected into the patient's body. Various fluid injection apparatus, which automatically perform such injection, have conventionally been proposed.

For example, Japanese Patent Laid-Open No. 2004-248734 has disclosed an apparatus in which injection condition can be set in consideration of a body-section of a patient (for example, a head part and a chest part) and an imaging-region (for example, organs such as a heart and a liver) as well as the patient's weight. US 2004/162484 relates to an injection apparatus comprising an injector head. Patent US 2007/238989 relates to management of information relating to medical fluids, containers therefor, and medical fluid administration devices for administering such medical fluids to patients.

### SUMMARY

### PROBLEMS TO BE SOLVED

According to the apparatus described in Japanese Patent Laid-Open No. 2004-248734, an optimal injection condition can be set up for each patient, thus waste of contrast medium can be reduced. Therefore it is significantly useful to set up an injection condition in consideration of a patient's body-section, an imaging region, a patient's weight and the like, furthermore, it would be desirable to facilitate an input for the setting.

The present invention has been made in view of the abovementioned problem, and it is an object thereof to provide a fluid injection apparatus in which injection condition can be set simply and the set contents can be reviewed easily.

### SOLUTION TO PROBLEM

To achieve the abovementioned problem, the present invention provides a fluid injection apparatus as described in the appended claims.

The term "body-section" indicates which section of a patient's body is to be imaged, and includes sections such as "head part", "chest part", "abdomen part", and "leg part" for example.

The term "imaging-region" refers to a region to be imaged (that is, a region to be enhanced), and includes organs such as "heart", "liver", "kidney", "blood vessel", and "tumor part" for example.

The term "selecting-region (selecting-region method)" refers to a method of setting injection condition associated with the selected item by selecting a body-section and/or an imaging-region.

The "fluid" refers to the contrast medium, physiological saline, or a mixture thereof.

A "user" refers to a person who uses a contrast medium injection apparatus, such as a doctor or other operator.

It is possible to provide the injector in which the injection conditions can be set simply and the set contents can be reviewed easily.

### BRIEF DESCRIPTION OF THE DRAWINGS

Fig. 1 is a perspective view of a fluid injection apparatus according to an embodiment of the present invention.
Fig. 2 is a perspective view showing mounting of a syringe on an injector head in Fig. 1.
Fig. 3A is a block diagram of a system in which the fluid injection apparatus in Fig. 1 is connected to an imaging apparatus.
Fig. 3B is a block diagram showing the configuration of a control part.
Fig. 4 is a diagram showing an image displayed on a display (setting of injection condition).
Fig. 5 is a diagram showing an image displayed on the display (setting of injection condition).
Fig. 6 is a diagram showing an image displayed on the display (setting of injection condition).
Fig. 7 is a diagram showing an image displayed on the display (setting of injection condition).
Fig. 8 is a diagram showing an image displayed on the display (review of injection condition).
Fig. 9a-9c are diagrams showing images displayed on the display (fluid injection).
Fig. 10 is a diagram showing an image displayed on the display (setting of injection condition).
Fig. 11 is a flow chart showing a procedure from the setting of the injection conditions to the injection of a fluid.
Fig. 12 is a diagram not according to the invention showing an image displayed on the display (home screen).
Fig. 13 is a diagram not according to the invention showing an image displayed on the display (emergency mode).
Fig. 14 is a diagram not according to the invention showing an image displayed on the display (emergency mode).
Fig. 15 is a diagram not according to the invention showing an image displayed on the display (emergency mode).
Fig. 16 is a diagram not according to the invention showing an image displayed on the display (pediatric mode).
Fig. 17 is a diagram not according to the invention showing an image displayed on the display (lean body weight mode).
Fig. 18 is a diagram not according to the invention showing an image displayed on the display (synchronization with the imaging apparatus).
Fig. 19 is a diagram not according to the invention showing an image displayed on the display (output of injection results).
Fig. 20 is a diagram not according to the invention showing other example of the image displayed in the pediatric mode.
Fig. 21 is a diagram not according to the invention showing an image displayed on the display (self check).
Fig. 22 is a diagram not according to the invention showing an image displayed on the display (time line of Timing Bolus Test injection).
Fig. 23 is a diagram not according to the invention showing an image displayed on the display (display of the state during the Timing Bolus Test injection).
Fig. 24 is a perspective view showing an example of an injector head display.
Fig. 25 is a block diagram of a system including the injector head display.

### DESCRIPTION OF EMBODIMENTS

An embodiment of the present invention will hereinafter be described with reference to the accompanying drawings. In the following, the embodiment of the present invention will be described in several separate sections.

### Section A: Injector in which injection condition can be set easily and the set contents can be reviewed easily

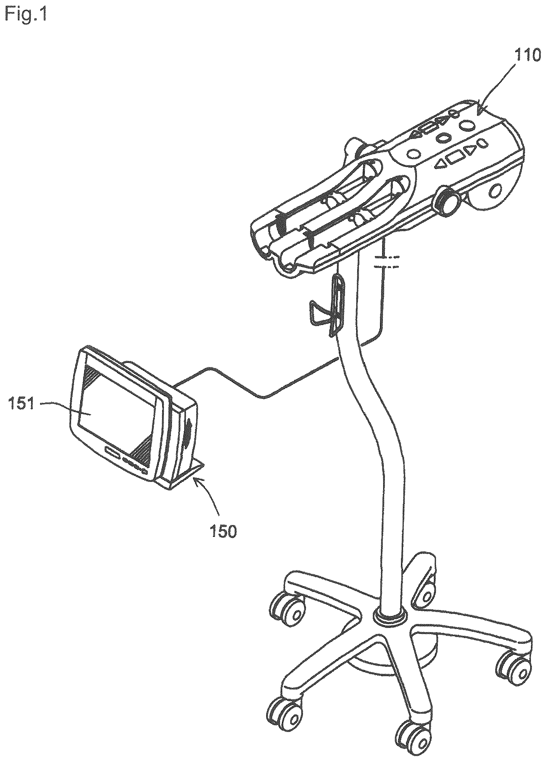

A fluid injection apparatus shown in Fig. 1 is used for injecting fluid into a patient. The injector includes an injector head 110 supported on a movable stand and a console 150 connected to the injector head 110. The console 150 is provided with a hand switch (not shown) manually operated by a user. The fluid injection apparatus may be used with, but not limited to, a CT scanner or MRI apparatus.

The injector head is of a dual type on which two syringes can be mounted. The injector head 110 shown in Fig. 1 is of the dual type on which syringe 200A filled with contrast medium and syringe 200B filled with saline are mounted.

In the following description, the syringes 200A and 200B may be referred to simply as "syringe 200" without making a distinction between them. The syringe 200 has a tubular cylinder member 210 and a piston member 220 slidably inserted thereinto.

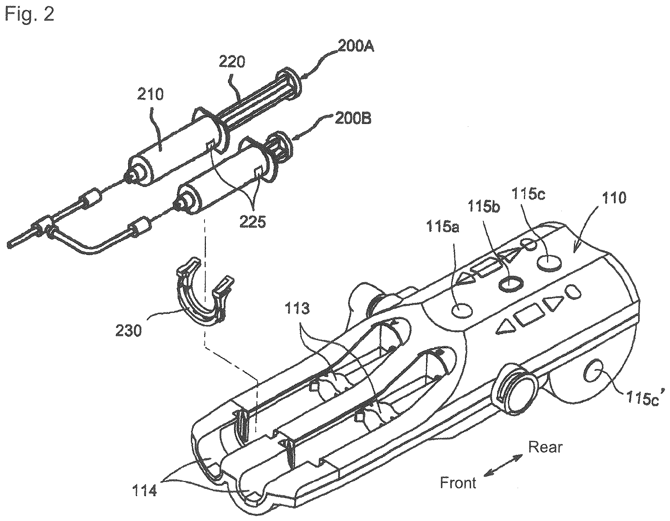

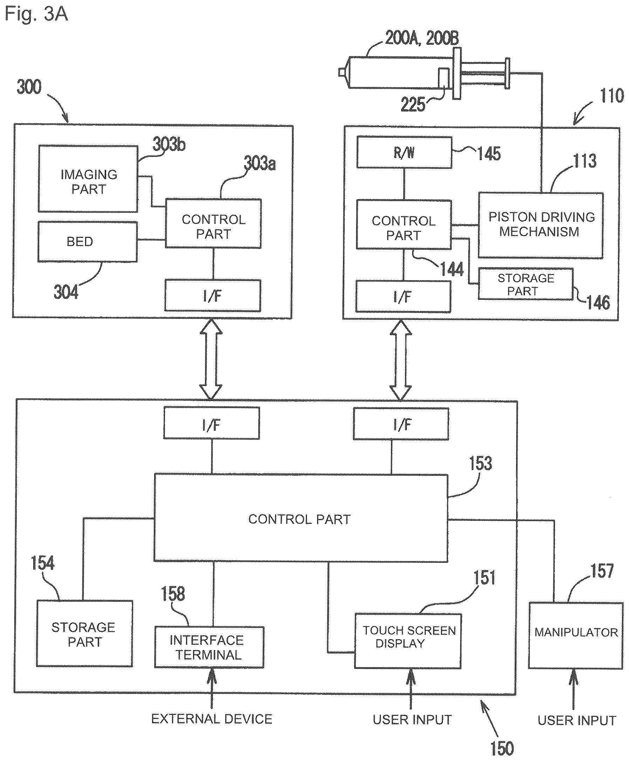

As shown in Fig. 2 and Fig. 3A, an IC tag 225 is attached to a portion of the cylinder member 210. The IC tag 225 holds information stored therein such as information relating to the syringe (including for example an identification information of the syringe, pressure limit of the syringe, inner diameter of the cylinder member, and stroke of the piston member) and information relating to the fluid filled in the syringe such as a product name, content information such as an iodine amount, an expiration date, and a fluid capacity. The IC tag may have a unique ID specific to the tag. The IC tag may have at least one of information items selected from a syringe size, a production number of the syringe, and a drug standardized code. An RFID (Radio Frequency Identification) tag may preferably be used as IC tag 225.

The injector head 110 has piston driving mechanisms 113 each of which configured to push the piston member 220 of the syringe 200. The piston driving mechanisms 113 are independently operable. Although not shown in detail, the piston driving mechanism 113 has a driving arm (syringe presser) that moves forward or rearward and a driving motor that serves as a driving source thereof. The piston driving mechanism 113 may contain a load cell (not shown) therein for detecting a force applied to push piston member 220. Result of detection of the load cell can be used for example so as to estimate the pressure of fluid during injection. Such a calculation of the estimated value may be performed in consideration of a size of a needle, a concentration of the fluid, or an injection condition etc.

Two syringe holding portions 114 in concave shape for holding syringes 200 are disposed in an upper forward surface of the injector head 110. Each syringe holding portion 114 may contain a reader/writer, not shown (see reference numeral 145 in Fig. 3A), for reading information of the IC tag 225 attached to the syringe. The reader/writer 145 may also have a function of writing some information onto the IC tag 225.

The syringe 200 may be, but not limited to, mounted on injector head 110 via an adapter 230 interposed between them as shown in Fig. 2. Each of syringes 200A and 200B may be mounted via the adapter 230, however, one adapter 230 is omitted in Fig. 2.

Some buttons 115a to 115c are disposed on an upper surface of the injector head 110. By way of example, the button 115a is a "check button" for enabling the fluid injection, the button 115b is a "start button" for starting fluid injection, and the button 115c is a "stop button" for stopping injection.

Another stop button 115c' may be disposed on a side surface of the injector head 110. Even if the injector head 110 is used at relatively high position, it will be easier to press button 115c' on the side surface of the head than button 115c on the upper surface. Such a configuration is advantageous with favorable operability. Light-emitting portions for emitting colored light may be disposed at a rear end of a housing of the injector head 110. Specifically, light-emitting portions may be disposed on the right side and the left side of the head respectively in association with each syringe. In this case, the pattern of light emission may be varied so as to indicate states of each syringe. The light-emitting portion for the contrast medium and the light-emitting portion for the saline may preferably emit light in different colors.

As shown in a block diagram of Fig. 3A, the injector head 110 has a control part 144 which controls the operation of piston driving mechanisms 113 and the reader/writer 145. The Injection head 110 also has a storage part 146 which temporarily stores information read from IC the tag 225, for example.

As shown in Fig. 1, the console 150 has a touch-screen display 151. The display 151 is configured to display, for example, an image for setting an injection condition and pressure of the fluid during injection (described below in detail). As shown in Fig. 3A, the console 150 has a control part 153 which controls an operation of each component, a storage part 154 which has various data stored therein, and an interface terminal 158 for connection to a predetermined external devices. The console 150 also has an interface for connection to the injector head 110 and an interface for connection to an imaging apparatus. The console 150 also has a controller 157 intended to be operated in a user's hand.

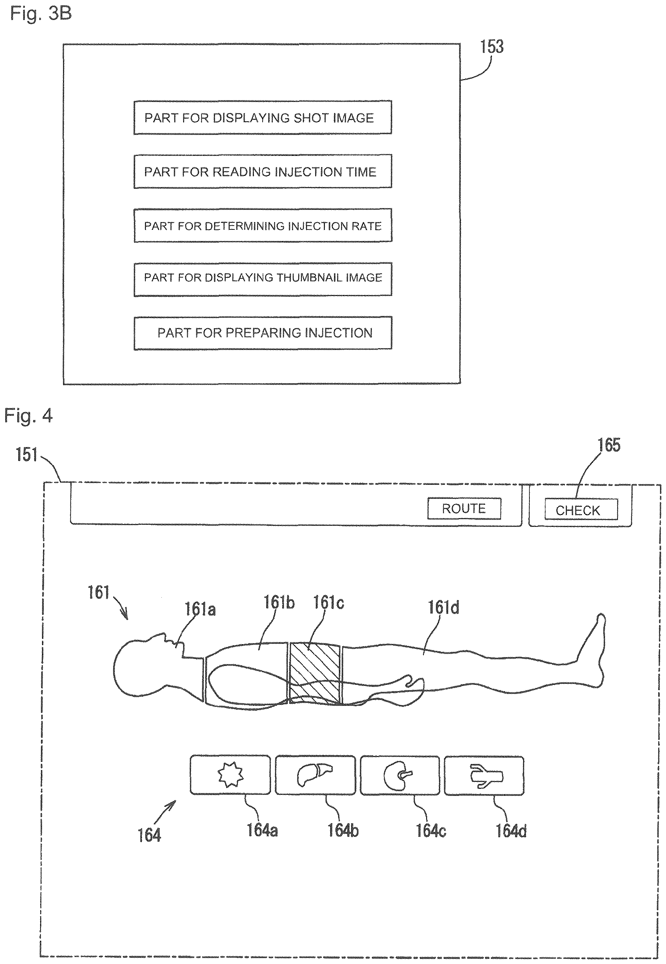

As shown in Fig. 3B, the control part 153 includes several parts in association with the functions (described later in detail) such as a part for displaying shot image, a part for reading injection time, a part for determining injection rate, a part for displaying thumbnail image, and a part for preparing (starting) injection.

The storage part 154 is configured to hold data of images to be displayed on display 151 stored therein, for example. The storage part 154 also holds algorithms including calculation expressions for setting injection conditions and data of an injection protocol stored therein. "Injection protocol" means which fluid should be injected in what amount at what flow rate. The injection rate may be constant or change over time. When contrast medium and saline are injected as in the present embodiment, an injection protocol also may include information relating to the sequence in which these fluids should be injected.

Data relating to injection protocols could be stored in storage part 154 in advance, or could be input from an external device connected through interface terminal 158, or could be input through an external storage medium inserted into a slot (not shown) of the console 150. Other configuration could be employed in which information about injection protocol is configured to be input from an external apparatus or system.

The Imaging apparatus 300 may be a CT scanner, an MRI apparatus, or an angiography apparatus, for example. The apparatus has an imaging part 303b which shoots diagnostic images of a patient, a bed 304 on which the patient lies, and a control part 303a which controls the operation of them, as shown in Fig. 3A.

The operation of the injection apparatus according to the present embodiment will hereinafter be described with illustration of specific images displayed on display 151.

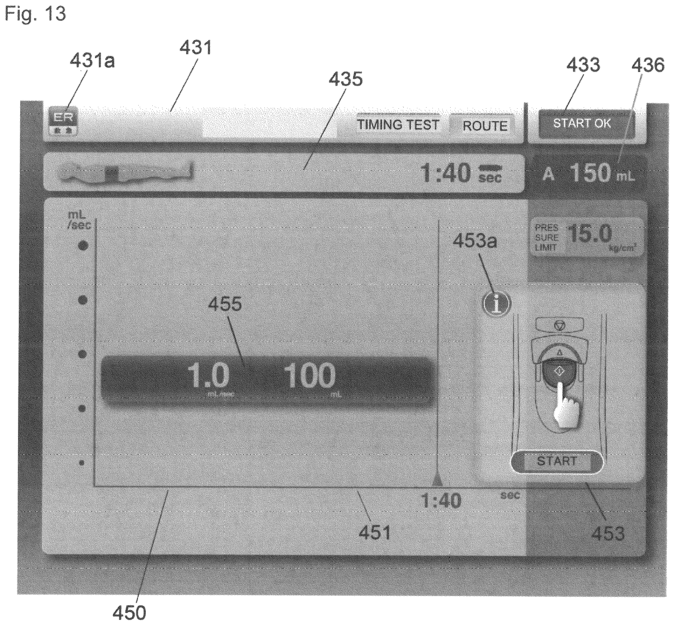

The injection apparatus according to the present embodiment is configured to display images such as for a normal fluid injection (see, for example Fig. 4 to Fig. 10), for injections in accordance with other injection methods (see, for example Fig. 13) or for editing. Two modes could be switched by pressing a hard button(s) disposed on the console 150. In this section, description is first made of the mode for performing the normal fluid injection with reference to Fig. 4 to Fig. 10.

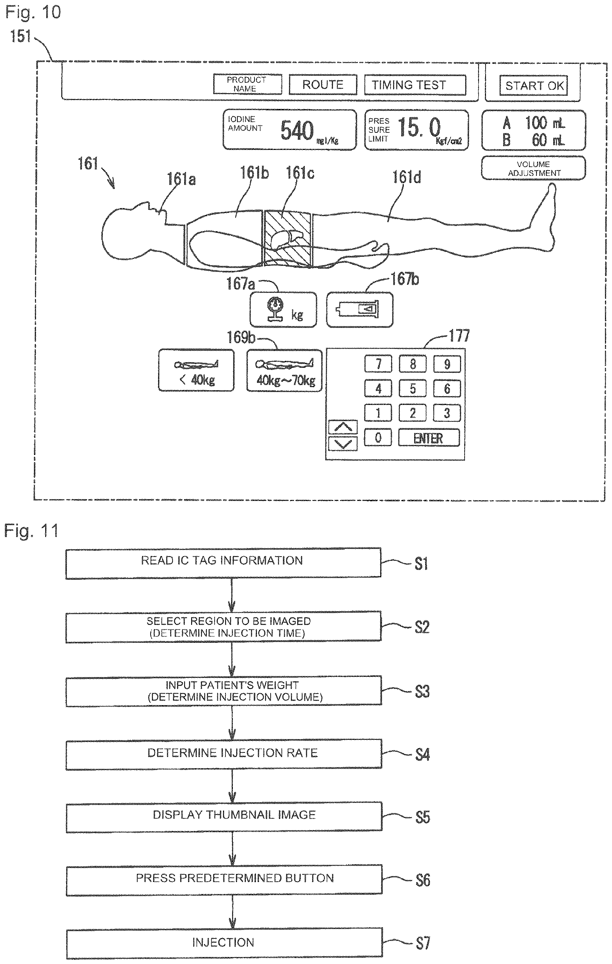

It should be noted that the following procedure is only illustrative and does not limit the present invention in any way. Fig. 11 is a flow chart showing the procedure from setting of the injection condition to fluid injection.

First, a user mounts syringes 200A and 200B on the injector head 110. The syringes 200A and 200B contain the contrast medium and the saline solution filled therein, respectively. When the syringe 200 is mounted, the data of the IC tag 225 will be read by the reader/writer 145 (Fig. 3A) of the injector head 110 (step S1). The read information may include, for example, information relating to the volume and the iodine concentration in the contrast medium, the product name of the contrast medium, and the pressure rating of the syringe. However, the information is not limited thereto, and various other types of information may be read.

For example, the volume of the contrast medium is 100 ml, 150 ml, or 200 ml. The iodine concentration is, for example, 240 mgI/ml, 300 mgI/ml, 320 mgI/ml, 350 mgI/ml, or 370 mgI/ml. The pressure rating of the syringe is for example 15 kgf/cm².

The term "pressure rating of the syringe" refers to a value of injection pressure above which the syringe would be damaged. In the apparatus of the present embodiment, the value may be automatically read from the IC tag on the syringe and set as a limit value for the piston driving mechanism, or, value input by the user may be set as a limit value. For example, if the value read from the IC tag on the syringe is 15 kg/cm² whereas the value input by the user is 20 kg/cm², the smaller value 15 kg/cm² would be automatically selected.

The information read by the reader/writer 145 is sent to the console 150 and then, as required, may be displayed on the display 151 of console 150.

### (Normal fluid injection mode)

Fig. 4 shows a screen displayed on the display 151. An image 161 representing a horizontally oriented human body shape is displayed at the center of the screen shown in Fig. 4. The human body image 161 is formed of multiple icons 161a to 161d in which a head part 161a, a chest part 161b, an abdomen part 161c, and a leg part 161d are shown. Each icon 161a to 161d is selected when a user touches it.

Fig. 4 depicts another group of icons 164 (described later in detail) below the human body image 161, however, the icons 164 are not displayed on an initial screen. Only the human body image 161 is displayed on the initial screen. In one embodiment, both the human body image 161 and the icons 164 may be displayed on the initial screen as shown in Fig. 4, however, according to the configuration in which only the image 161 is displayed, items displayed on the screen can be simpler, to thereby help user manipulate the screen intuitively.

When the user touches one of the icons (for example, abdomen part 161c) of human body image 161, icons 164a to 164d representing imaging-regions will be displayed in association with the abdomen part (in this case, "tumor part," "liver," "kidney," and "blood vessel").

On the screen shown in Fig. 4, the icons 164a to 164d are displayed below the human body image 161, being aligned horizontally. Each icon 164a to 164d includes only a graphic image representing an organ, and does not include characters such as "tumor part," "liver," "kidney," and "blood vessel", in this example. These characters may or may not be displayed. According to the configuration in which the icon includes only the graphic image without characters as shown in Fig. 4, contents on the display become simple. Preferably, the screen may toggle the display between such characters.

In the state shown in Fig. 4, the abdomen part 161c is highlighted in the human body image 161. This allows the user to recognize that this body-section has been selected.

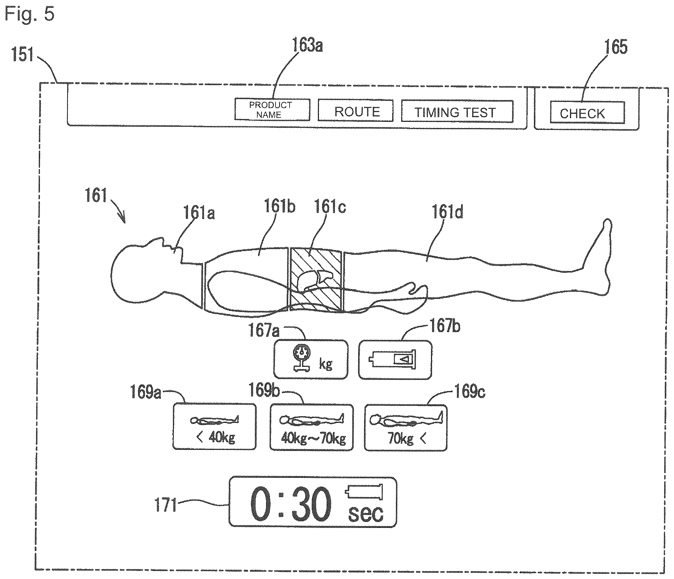

Next, an imaging-region is selected (step S2). By way of example, the user touches the icon 164b of the liver on the screen shown in Fig. 4. Then, the graphic image of the liver may preferably be moved as if it was taken into the abdomen part 161c of the human body image 161 and then be displayed inside abdomen part 161c as shown in Fig. 5.

Once an imaging-region is selected in this manner, a fluid injection time associated with imaging-region will be read from storage part 154 and be displayed on the display 151. In this example, the injection time associated with the liver is 30 seconds, and an icon 171 showing "0:30 sec" is displayed in a lower portion of the screen.

On the screen in Fig. 5, icons 169a to 169c for selecting patient's weights are displayed on the display 151. The icons 169a to 169c are placed horizontally in a line below human body image 161. Weight for patients includes three classifications. The icon 169a represents weight less than 40 kg, the icon 169b represents weight no less than 40 kg and no greater than 70 kg, and the icon 169c represents weight greater than 70 kg, respectively.

The number of classifications of the weights may be changed. The weight value in each classification may also be freely changed. Although a unit of weight "Kg" is displayed in the icons in Fig. 5, the unit may not necessarily be displayed, namely, the icons may include only values such as "40 to 70."

On the screen in Fig. 5, two icons 167a and 167b are displayed between the icons 169a to 169d and the human body image 161. The icon 167a shows a graphic of a scale. The icon 167b shows a graphic of the syringe. By touching the icon 167a of scale, a user can input more specific patient's weight (described later in detail). By touching the icon 167b of syringe, a mode may be switched to a "flow rate mode" where an injection rate can be selected freely (described later in detail). The icon 167b could be displayed on the screen in Fig. 4 rather than in Fig. 5, further, the icon 167b could be displayed on the side of the icon 164d.

Next, the user touches one of three icons 169a to 169c on the screen in Fig. 5 so as to input the patient's weight (step S3). In the following, description is made of an example in which the user touches icon 169b representing weights no less than 40 kg and no greater than 70 kg.

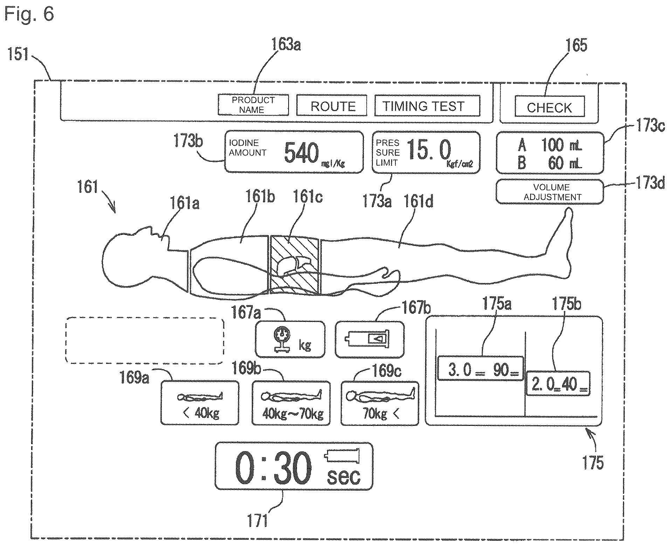

Although not shown in Fig. 6, when the weight classification is selected, the selected icon 169b may be highlighted. As an example of the display form, the icon 171 representing the injection time may be moved diagonally upward from the lower portion on the screen to near a position indicated by a broken line on the left of the icon 167a of scale and then be displayed there.

On the screen in Fig. 6, information read from the IC tag 225 is displayed. For example, the pressure limit of the syringe is displayed as "15.0 kgf/cm²" in the icon 173a. The iodine amount per unit weight required to enhance a liver is displayed as "540 mgI/kg" in the icon 173b. These icons 173a and 173b are displayed above human body image 161.

On the screen in Fig. 6, an icon 173c representing injection volume of the fluid is displayed. Specifically, "A 100 ml" and "B 60 ml" are displayed. This means that the injection volume of the contrast medium is 100 ml, whereas the volume of the saline is 60 ml. An icon 173d showing "Volume Adjustment" is displayed below the icon 173c.

When the volume of fluid remaining in the syringe (for example, 70 ml) is smaller than the set volume of injection (for example, 76 ml), the user presses icon 173d to set the volume of the remaining fluid as the injection volume.

It should be noted that, while the specific values of fluid volume, injection rate, concentration and the like have been depicted on the screens in Fig. 5 to Fig. 10, they are merely illustrative and do not limit the present invention in any way.

Next, a procedure of determining the injection volume of the contrast medium is described. In the present embodiment, the injection volume is calculated on the basis of (i) a patient's weight, (ii) an iodine concentration in the contrast medium, and (iii) an iodine amount required per unit weight associated with the imaging-region. In particular, a calculation may be performed assuming that the patient's weight is 55 kg which is an intermediate value between 40 kg to 70 kg. The total iodine amount to be injected to the patient is calculated on the basis of weight and required iodine amount. Then, a contrast medium volume to be injected is calculated on the basis of the total iodine amount and the iodine concentration in the contrast medium.

Although the representative value of the classification from 40 kg to 70 kg is 55 kg in the example, this value may be changed in an initial setting. In addition, the range of the weight classification (for example, the classification from 40 kg to 70 kg) itself be changed in the initial setting.

In a conventional fluid injection apparatus, the determination of the contrast medium volume made in consideration of the patient's weight typically requires user to input, for example data relating to a patient's weight and contrast medium information manually, which involves complicated operations. On the other hand, according to the present apparatus, the weight information can be input only by touching one icon 169b (by way of example) and the contrast medium information can be read from the IC tag 225, resulting in extremely simple operation.

Next, description is made of the calculation of the injection rate of the contrast medium (step S4). In the apparatus according to the present embodiment, the injection time could be determined as "0:30 sec" by merely selecting the selection of liver 164b, for example. The injection rate of contrast medium will be calculated automatically, for example by a calculation dividing the contrast medium volume calculated at the abovementioned step by the selected injection time.

Through the series of steps described above, the injection time [sec] of contrast medium, the injection volume [ml] of contrast medium, and the injection rate [ml/sec] of contrast medium are calculated, and then the injection condition of the contrast medium is determined. Since the injection condition of saline can be determined by a known method, detailed description is omitted.

As shown in Fig. 6, the injection condition of contrast medium determined in this manner and the injection condition of saline will be displayed as a thumbnail image 175 (step S5). Alternatively, the thumbnail image 175 may be displayed without performing step S3 or S4. For example, a configuration can be employed in which a default weight classification will be selected and an injection rate associated therewith will be determined when selecting one imaging-region at step S2, and then the thumbnail image corresponding to them will be automatically displayed.

Thumbnail image 175 includes a graph in which the horizontal axis represents an elapsed time and the vertical axis represents the injection rate. Two condition images 175a and 175b may be displayed within the graph. Specifically, the condition image 175a shows the injection conditions of contrast medium, whereas the condition image 175b shows the injection conditions of saline. Each image 175a or 175b is displayed at the position of a height corresponding to the injection rate and has a horizontal width corresponding to the injection time.

A conventional apparatus typically displays information relating to the injection time, the injection rate, and the injection volume simply as numeric values at the time of setting of the injection condition. Thus, it is difficult to review the set injection conditions intuitively. According to the apparatus of the present embodiment, however, user can check the injection condition easily by looking at the thumbnail image 175.

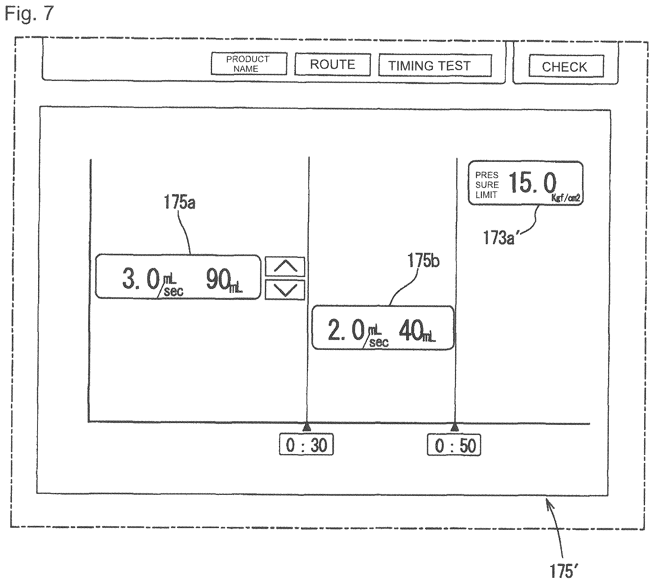

It would be contemplated that a set injection condition is displayed as a larger graph 175' as shown in Fig. 7 (described below in detail). However, In this case, space for displaying the various icons (for example, 171, 169b, and 161) for setting the injection condition may become insufficient. By contrast, according to the configuration in which the graph is displayed as the thumbnail image 175, the icons 171, 169a to 169c, 161 and the like can be displayed without any changes. User can modify the injection condition easily by using such icons, if necessary.

It is noted that the horizontal width and/or height of the condition image in the thumbnail image may be changed in accordance with the set conditions, or may not be changed.

Referring again to Fig. 6, when the user touches the thumbnail image 175 on the screen in Fig. 6, the image could be enlarged and then the larger image 175' is displayed as shown in Fig. 7. The image 175' includes the condition image 175a of contrast medium, the condition image 175b of saline, and a syringe pressure limit image 173a'.

A configuration is employed in which the injection rate will be changed when the user moves the image 175a upward or downward. The condition image could also be changed by touching a "up" button or a "down" button.

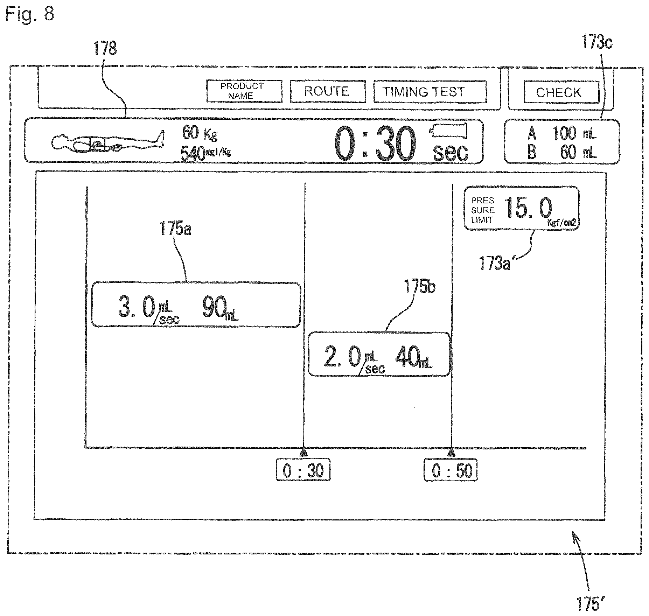

Fig. 8 shows a screen for finally reviewing the injection condition. On this screen, an image 175' of a graph for injection condition is displayed, in a large size, at the center of the screen. A horizontal window 178 is positioned above the image 175'. Information of the patient's weight, iodine amount required per weight, injection time and the like is displayed in the window 178. Icon 173c is positioned on the right of the window 178, allowing user to check the volume of contrast medium (A) and saline (B).

Other information item such as the selected weight classification may be displayed in the window 178. The iodine amount per weight may be displayed, and the iodine amount per weight and time may be displayed.

After checking the injection conditions on the screen in Fig. 8, the user presses a button 165 on the display or a hard button on the injector head (step S6). Then, the apparatus will start a state in which the fluid injection can be started. The fluid injections may be conducted in the order of the contrast medium and then the physiological saline in this example (step S7).

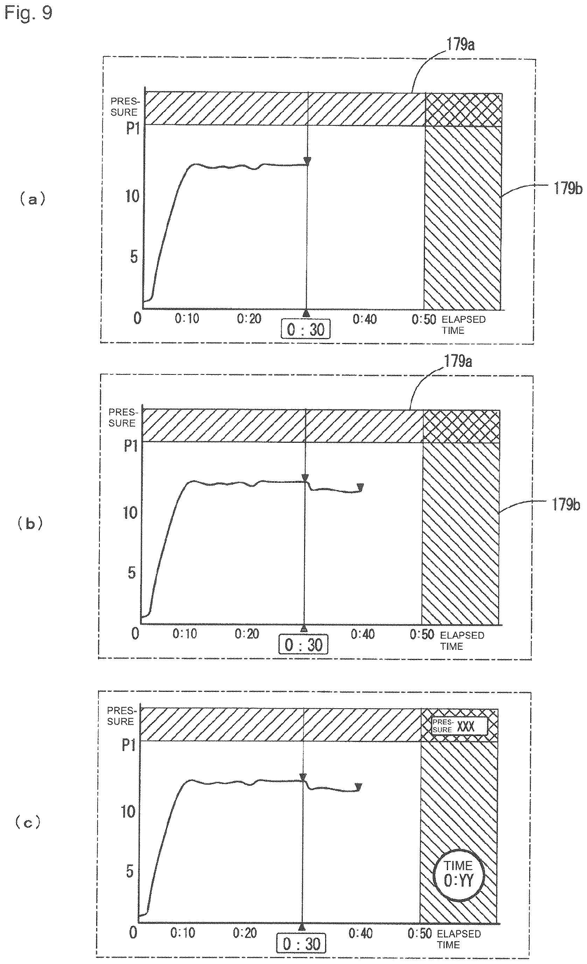

Once contrast medium injection is started, a pressure graph will be displayed on the display as shown in Fig. 9(a). This graph shows the pressure of the fluid during the injection in real time, in which the horizontal axis represents the elapsed time and the vertical axis represents the pressure. The pressure of the fluid may be calculated, for example, on the basis of a pressure with which the piston driving mechanism pushes the piston member of the syringe. The pressure with which the piston driving mechanism pushes the piston member can be detected by using a load cell of the piston driving mechanism, for example.

Fig. 9(a) shows a graph at the time when a certain time has elapsed after the start of the injection, and Fig. 9(b) shows a graph at the time when some time has further elapsed after the graph in Fig. 9(a). In these graphs, a region above a limit value P1 is displayed in a predetermined color or displayed in a predetermined pattern so as to allow easy checking whether the fluid pressure exceeds P1 or not. If the fluid pressure exceeds the limit value P1, an alarm may preferably be issued to the user. The alarm could be a message with sound or a message with image or characters. Further, it is preferable that an output power of the piston driving mechanism will be automatically adjusted so as to reduce the pressure of the fluid. According to the abovementioned configuration, an increase in the fluid pressure is prevented, as a result, damage to the syringe or the like can be prevented.

A region 179b that exceeds a predetermined time may be displayed in a color or a predetermined pattern in the graph shown in Fig. 9, similarly to the above region. As shown in Fig. 9(c), an image for indicating information relating to the pressure and/or elapsed time could be displayed within regions 179a or 179b.

Fig. 10 shows a screen for inputting a patient's weight in detail. As shown in Fig. 10, when the user touches icon 169b of the selected weight classification on the screen in Fig. 6 in the apparatus according to the present embodiment, a keypad 177 will be displayed. The user can manually input (by way of example) values through the keypad 177 so as to input specific patient's weight. For example, if "62 kg" is entered, icons 169a to 169c will be disappear and another icon for representing "62 kg" will be displayed.

The scale icon 167a and syringe icon 167b displayed on the screen in Fig. 10 are used to switch modes for setting injection conditions. When the icon 167a is selected, a "weight input mode" (the abovementioned series of setting steps) will start. This mode may be set as a default mode in the present embodiment. When the icon 167b is selected, "flow rate mode" will start. In the weight input mode, the injection time (for example, 0:30 sec) will have high priority, and the injection rate will be calculated on the basis of that injection time. On the other hand, in the flow rate mode, an injection rate can be set freely by user such as 1.5 ml/sec or 2.0 ml/sec. This mode can be used when the injection rate is an important factor for the image visualization, for example.

With respect to a pressure graph in Fig. 9, it is possible to plot not only the detected real-time pressure of the fluid but also an ideal pattern which may be achieved if the injection can be performed on the injection condition. The pattern may be displayed either as a line or a band having a certain width.

The series of operations described above basically includes the following processing:
(a) processing of displaying the images of the plurality of imaging-regions on the display;
(b) processing of, when one of the imaging-regions is selected, reading the injection time associated with the imaging-region;
(c) processing of determining the contrast medium volume to be injected into the patient;
(d) processing of determining the injection rate of the contrast medium based on the determined contrast medium volume and the injection time;
(e) processing of displaying the graph of the injection condition showing the relationship between the injection time and the injection rate as the thumbnail image on the display; and
(f) processing of, when the predetermined icon on the display and/or the predetermined button on the injector head is pressed thereafter, starting a state in which injection can be performed in accordance with the injection condition. Each processing can be performed by each processing part of control part 153.

According to the present invention configured as described in the claims, when the injection condition is set, the condition will be displayed as the thumbnail image 175 (see Fig. 6) on display 151. Thus, the user can check the condition through the thumbnail image 175. Since the image is displayed as the relatively small thumbnail image rather than the large image as shown in Fig. 7, the icons for setting injection conditions (for example, 171, 169a to 169c, and 161) can remain at the initial positions without any move, therefore the icons can be used to make modifications easily.

In addition, according to the fluid injection apparatus of the present embodiment, the injection condition can be set up through the simple steps of; (i) mounting syringe(s) on the injector head, (ii) selecting an imaging-region, and (iii) selecting a patient's weight classification, in principle. To realize such a simple setting, the injection time of the contrast medium (for example, "0:30 sec") will be preferably determined in association with the selected imaging-region, for example at the time of that selection. To lessen time and effort for manually inputting information concerning the contrast medium, it may be preferable that data of iodine concentration in the contrast medium, contrast medium volume and the like can be read automatically from the IC tag 225 on the syringe.

User also can input data of patient's weight by inputting specific numeric value through a numeric keypad. Whereas, according to the above embodiment in which one of the icons 169a to 169c (Fig. 5) is selected for input, burden to the user can be reduced.

In the apparatus of the present embodiment, the injection time of contrast medium is determined based on the selected imaging-region (0:30 sec in the example of Fig. 5), and then the injection time will remain the same in principle regardless of the patient's weight. According to such a manner, the same injection time will be used in performing several diagnoses with respect to one patient (for a common imaging-region), or, in performing diagnoses on a plurality of patients (for a common imaging-region), thereby eliminating needs to adjust the timing to start scanning in a CT apparatus or the like.

The present invention is not limited to the above, and the following may be used. For example, to use syringe 200 without IC tag 225 the user may touch a product name icon 163a of on the screen (for example, Fig. 5) to display a pull-down list and select one of products in the list to input the information of the product name, the iodine amount and the like.

In the apparatus of the present embodiment, the predetermined injection times such as 30, 45, or 60 seconds, have been registered for each of the imaging-regions (for example, the tumor part, liver, kidney, and blood vessel). Such data may be stored on storage part 154 of console 150 (see Fig. 3A), or may be stored on an external device and read to console 150 through a network.

Further, a device that can measure patient's weight or a device that holds information of patient's weight could be connected to console 150. In that case, weight information may preferably be input from that device to make the automatic selection of the classification of the patient's weight (see icons 169a to 169c in Fig. 5). This configuration enables omission of the step of selecting a patient's weight.

It is noted that two icons or four or more icons could be displayed for weight icons. Alternatively, instead of selection of the weight classification, one default weight (for example, 60 kg) may be automatically selected.

To start fluid injection, a configuration can be employed in which a certain button such as an icon on the screen and/or a button on the injector head will be selected in the state of Fig. 6 in order to start an injection mode, without displaying image 175' in Fig. 7 or Fig. 8. By way of example, a configuration can be employed in which the button 165 or the hard button on the injector head is pressed in the state of Fig. 6, then an indication in the button 165 will change to "start OK". This indication represents a state in which the injection can be started.

While Fig. 4 to Fig. 10 show horizontally oriented human body image 161 as an example, the apparatus according to the present invention may display a vertically oriented human body image. The number of the body-section included in human body image 161 is not limited to four but may be three or less or five or more.

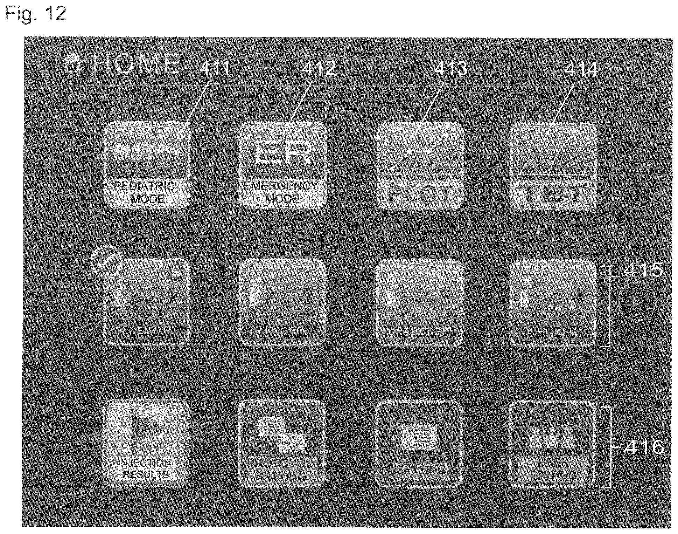

What is following does not form part of the invention but represents background art that is useful for understanding the invention. Next, description is made of examples of the image displayed on display 151 with reference to Fig. 12 to Fig. 23.

### Section B: Contrast medium injector in which injection mode and the like can be set or changed simply on console to achieve injection in various methods (Mode for selecting another injection method or performing predetermined editing)

It is described according to the present section to provide a contrast medium injection apparatus in which modes for injection and/or modes for setting injection protocol (hereinafter referred to simply as the "injection mode and the like") can be set or changed easily on the console to conduct an injection in various methods. It is also an object to provide a contrast medium injection apparatus in which the injection mode and the like can be set up in accordance with a preference of a doctor when some doctors use the same injector.

Fig. 12 shows a "home" screen. The home screen may be displayed for example by selecting a hard button disposed on the console. Alternatively, the home screen could be displayed by pressing some icon or the like on the screen, or the home screen could be automatically displayed after a predetermined time (i.e. idle time) has passed.

On the home screen in Fig. 12, icons are placed in a matrix arrangement, including three rows and four columns, for example. Each of the icons has a generally square shape with rounded corners in this example, however, the icon may have another shape such as a rectangle, a polygon, and a circle. The number of the icons displayed on the home screen is not limited to a specific number. However, when the screen includes too many icons, user may have difficulty with intuitive manipulation. Thus, for example, the number of icons is preferably set such that the number of rows ranges from 3 to 5, whereas the number of columns ranges from 3 to 6 (that is, a 3 by 3 matrix to a 5 by 6 matrix). According to such configurations, information can be displayed sufficiently without compromising operability.

As shown in Fig. 12, four icons 411 to 414 are displayed side by side in an upper level on the home screen. The icons 411 to 414 are used for "pediatric mode," "emergency mode," "plot injection mode," and "test bolus tracking (TBT) injection mode," respectively.

In an intermediate level on the home screen, four icons 415 are displayed for selecting one of doctor 1 to 4 who should use this contrast medium injection apparatus. The number of icons 415 is not limited particularly, but for example, only one, two, or three icons may be displayed.

In a lower level on the home screen, some icons 416 are displayed for starting various functions of the contrast medium injection apparatus. In this example, icons such as an icon for displaying results of fluid injection, an icon for protocol setting, an icon for changing settings, and an icon editing users (such as a doctor).

The position of each icon on the home screen may preferably be changeable. In principle, it may be possible to rearrange the icons, and to change display/not-display of each icon freely. However, some icons may preferably be configured to remain displayed all the time on the screen (i.e. they can not be set to not-display). For example, the emergency mode icon 412 can be set to be displayed all the time so that fluid injection in an emergency can be conducted properly.

Next, functions of each of the icons are described. In the following, description is first made of the "emergency mode" and then of the "pediatric mode" and another mode.

### (Emergency mode)

The "emergency mode" refers to a mode that helps other doctor to perform contrast medium injection promptly even when a particular doctor is absent due to for example nighttime diagnosis. When the emergency mode icon 412 is selected on the home screen in Fig. 12, the emergency mode will be started, and a screen as shown in Fig. 13 will be displayed on the display upon a input by doctor (described below in detail).

A status bar 431 is displayed in an upper portion on the screen. An ER icon 431a indicating that the emergency mode is being currently selected and several other icons are displayed in the status bar 431.

The "other icons" include, for example, a "route" icon for performing an operation for checking that an patency route to a patient is properly established before fluid injection, and an icon for performing a conventionally known "timing (bolus) test." Further, although not shown, an identification mark for indicating a product name and/or manufacturer of fluid in the syringe could also be displayed in the status bar 431. Start button 433 is displayed on the right of status bar 431.

A large window 450 is displayed at the center of the screen. In this window, an injection condition image 451 of the emergency mode and a guidance image 453 for showing some guidance to users are displayed

The injection condition image 451 is a graph in which the vertical axis represents the injection rate of the fluid and the horizontal axis represents the injection time. A condition bar icon 455 is displayed in the graph. Since it is important to enable users to set up injection condition quickly in an emergency, the condition bar icon 455 includes an injection condition with a preset injection rate and preset injection time (for example, a rate of 1.0 ml/sec and an amount of 100 ml). The injection conditions may be changed by the user or may be made unchangeable.

The guidance image 453 may provide the user with information relating to how to input through the injector head and/or the console. In the example of Fig. 13, the guidance image 453 shows, as an image, information indicating that user should press a manipulator device of the console (i.e. hand switch, not shown in Fig. 1).

It is noted that the guidance image 453 could be displayed after pressing the "i" icon 453a. In another aspect, the guidance image 453 may automatically appear after the elapse of a predetermined time without pressing of "i" button 453a.

In Fig. 13, a horizontal display bar 435 is displayed between the window 450 at the center of the screen and the status bar 431 in the upper portion of the screen. For example, a body image of the patient and a fluid injection time are displayed in the display bar 435. For example, the body image of the patient is formed of a plurality of body-sections in which the selected body-section (described below in detail) may be highlighted. This allows user to know visually which body-section has been selected.

A display part 436 for indicating fluid volume in the syringe is displayed on the right of horizontally oriented information display bar 435. In this example, "150 ml" is displayed, indicating that the volume of contrast medium in the syringe is 150 ml.

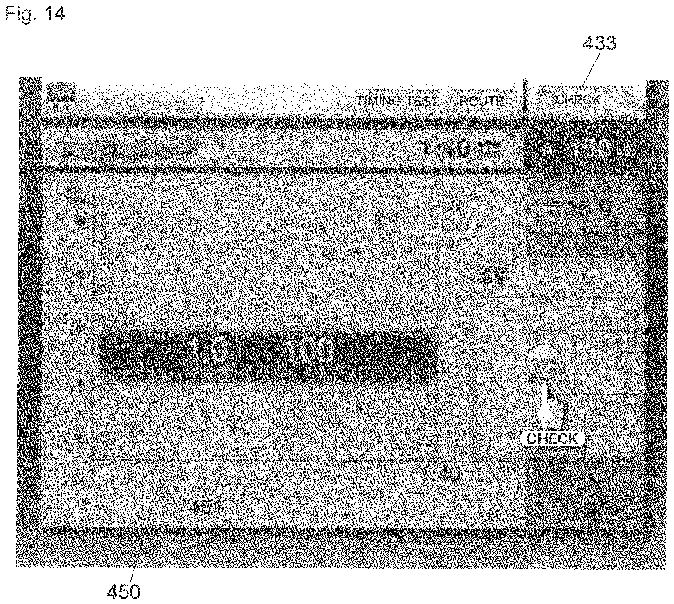

When a user presses the start button 433 on the screen or the manipulator (not shown) of the console after reviewing the injection conditions and the like on the screen in Fig. 13, a screen as shown in Fig. 14 will be displayed. On this screen, the indication in start button 433 has been changed to "check", and instruction within the guidance screen 453 also has been changed. Specifically, the guidance image 453 shows, as an image, information indicating that the user should press check button 115a (see Fig. 2) on injector head 110. Next, the user presses the check button 115a or the start button 433 to start fluid injection in the emergency mode.

According to the configuration of this embodiment, when a user (1) selects the emergency mode on the home screen in Fig. 12, (2) makes entry of "start OK" on the screen or the console after confirming the injection condition, and then (3) presses some button on the injector head (by way of example), fluid injection can be started safely and quickly in the emergency mode.

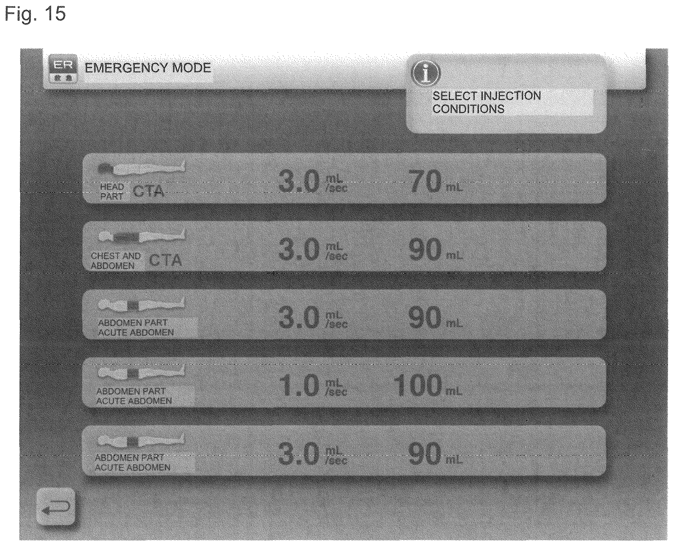

Alternatively, in performing the emergency mode, a screen as shown in Fig. 15 may be first displayed to allow selection of one of several options for injection condition. In the example of Fig. 15, "head part CTA," "chest and abdomen part CTA," "abdomen part acute abdomen (condition 1)," "abdomen part acute abdomen (condition 2)," and "abdomen part acute abdomen (condition 3)" are displayed. According to the configuration, preferable contrast medium injection can be performed in consideration of the region and the patient characteristics even in the emergency mode. When one of the candidates for injection condition is selected, the screen in Fig. 14 may be displayed, for example.

In addition to the above examples, other configurations may be employed which display screens as follow. For example, when the emergency mode is selected on the home screen in Fig. 12 similarly to the above case, the screen as shown in Fig. 15 will be displayed. When one option for injection condition is selected on the screen in Fig. 15, the screen in Fig. 14 will be displayed. Next, when check button 433 on the screen in Fig. 14 or the check button on the injector head is pressed, the indicator in the icon 433 will be changed from "check" to "start OK" (Fig. 13). Finally, in the state of Fig. 13, the start button on the injector head or the switch in controller 157 is pressed to start the injection. Various embodiments for displaying images on the display may be used.

### (Child mode)

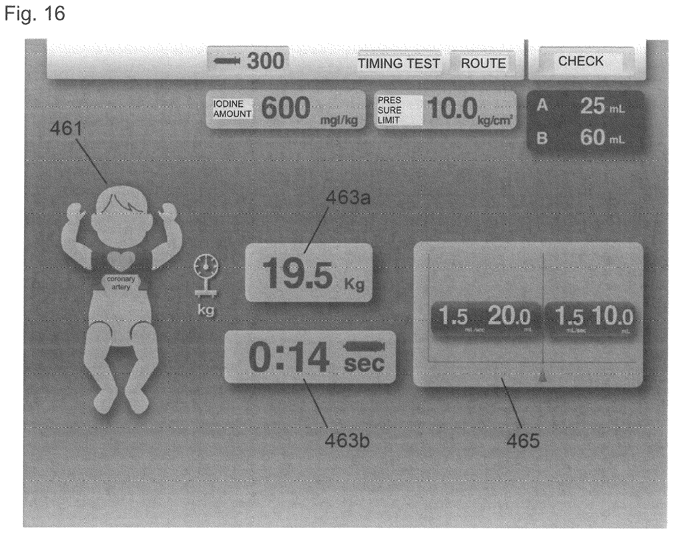

Once the pediatric mode icon 411 is selected on the home screen (Fig. 12), the pediatric mode will start and a screen as shown in Fig. 16 will be displayed on the display. An image of vertical human body(by way of example) representing a child is displayed in a left portion of the screen. The human body image 461 includes some icons indicating selectable body-sections (for example, a head part, a chest part, an abdomen part, and a leg part). Fig. 16 shows a state in which the "chest part" has been selected and the selected section is highlighted.

In association with the selected body-section, a predetermined injection condition will be displayed in the thumbnail image 465. By way of example, the thumbnail image 465 shows an injection condition for injecting i) 20 ml contrast medium at 1.5 ml/sec and ii) 10 ml saline at 1.5 ml/sec. A weight icon 463a for setting weight of child and a injection time icon 463b indicating injection time are displayed at the center of the screen in Fig. 16.

According to the configuration of the present embodiment as described above, injection condition suitable for each patient, not only for adults but also for children, can be set up. In the pediatric mode, it is noted that options for several injection conditions may also be displayed as shown in Fig. 15. However, children generally may not require such a detailed setting compared with adult patients, therefore such a function may be omitted to enable quicker and simpler setting.

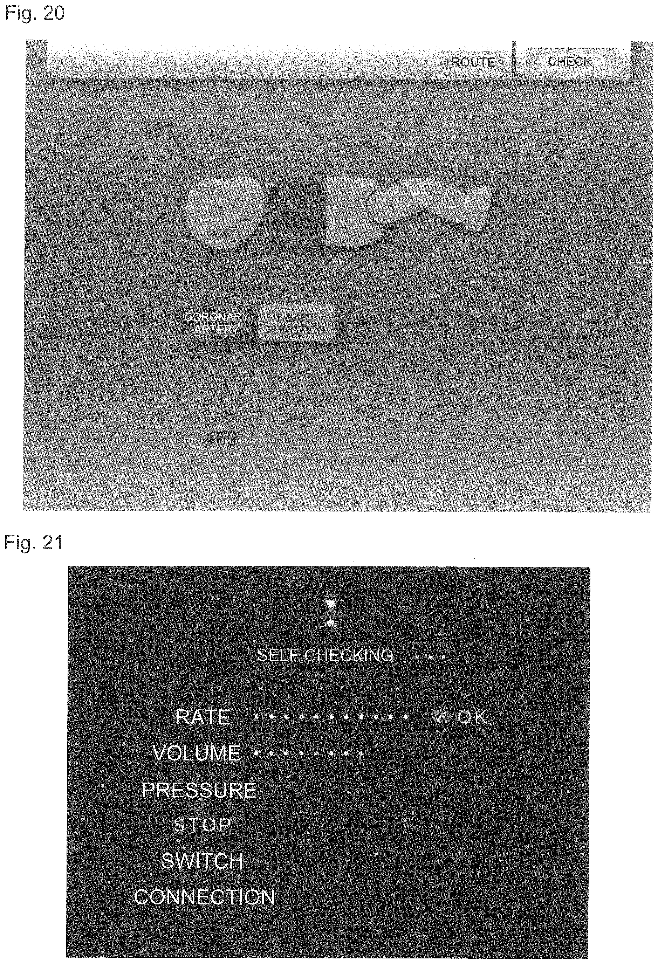

It is noted that the human body image 461 of the child is not limited to the vertically oriented one; a horizontally oriented one could be employed. In particular, a human body image 461' of horizontally oriented child as shown in Fig. 20 could be displayed. Fig. 20 shows a screen in which one of body-sections (chest part) has been selected from human body image 461' and imaging-region icons 469 ("coronary artery" and "heart function" by way of example) associated with the section is displayed. The number of the sections included in the human body image is not limited particularly. It is also noted that the pediatric mode could be started for example by mounting an adapter for small syringes (for example 20 ml) on the injector head. Specifically, the screen may be automatically switched to the image of human body image 461' of the child as shown in Fig. 20 when the adapter is mounted, for example.

### (Other injection modes)

When the "plot injection" icon 413 or "test bolus tracking" injection icon 414 on the home screen in Fig. 12 is selected, a plot injection mode or a test bolus tracking injection mode will be started. Detailed description of these injection modes is omitted since they are conventionally known. According to the configuration of the present embodiment, the user can intuitively select the various injection modes on the home screen, so that time and effort of the user in setting the injection conditions can be reduced.

It is noted that icon(s) for other condition setting methods (for example, a weight input mode, a lean body weight mode, a body surface area mode, and a blood volume mode) can preferably be displayed on the home screen so as to meet preferences of the users.

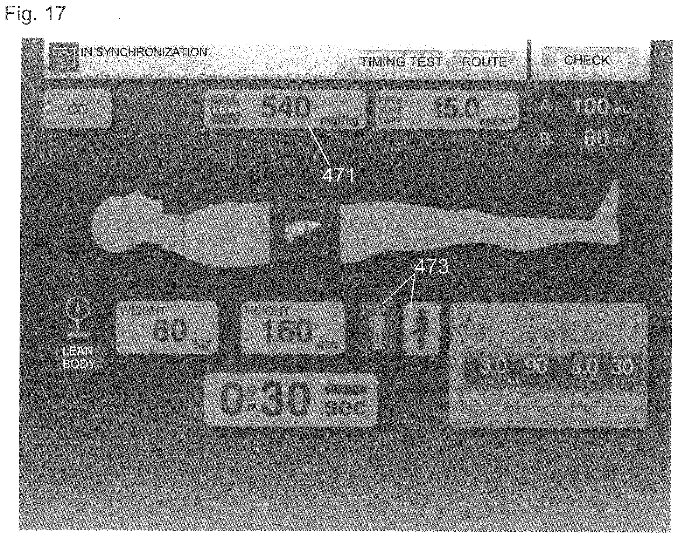

While a conventionally known method can be used for setting these injection modes/conditions, a screen as shown in Fig. 17 may be displayed as the "Lean Body Weight" mode, for example. On this screen, icons 473 for selecting whether a patient is a man or a woman is displayed in addition to a patient's weight icon, a height icon, a fluid information icon, and an injection condition icon. Window 471 includes a character "LBW" that indicates the lean body weight mode have been selected, as well as an iodine amount per weight (540 mgl/Kg).

In each of the injection modes, a predetermined alarm as conventionally known may be issued to the user. For example, the alarm may be issued when the set injection rate, injection amount, or injection pressure is higher or lower than a predetermined reference value. In this case, the associated operation may be forcedly stopped in addition to the issue of the alarm.

### (Doctor selection icon)

As shown in Fig. 12, a doctor who should use the injector can be selected with the icon in the present embodiment. Fig. 12 shows four doctors (users 1 to 4) by way of example. A check mark on the icon of user 1 indicates that this doctor is a default doctor (doctor 1 would be selected when the doctor selection is skipped).

According to the present embodiment, each doctor is able to previously set up injection conditions in accordance with their preferences. It is possible to set in detail (i) by way of example, which imaging-region should be displayed in response to pressing of each of the body-sections in the region selection mode as shown in Fig. 4, (ii) what period of injection time and what injection rate are set, and/or (iii) which injection pattern is used, and the like.

According to the configuration, the doctor can use the condition setting mode to meet his preference.

Of course, other items (for example, which icon is displayed on the home screen, in what arrangement the icons are displayed, and the like) may be freely changed to meet the preference of each doctor. For example, the icons displayed in the top level on the home screen in Fig. 12 may be displayed in the intermediate level, while the icons displayed in the intermediate level may be displayed in the top level.

On the other hand, some icons may be configured such that they cannot be deleted from the home screen. For example, when an emergency mode is considered as relatively important in a hospital, the icon for emergency mode is displayed without fail on the home screen as described above and only the other icons can be changed to preferable icons as described above.

### (Icons for performing various functions and the like)

The "protocol setting" icon on the home screen (Fig. 12) is provided for newly setting an injection protocol and/or changing existing injection protocols. The injection protocol is not limited particularly, various methods are preferably set such as a protocol called variable injection in which the injection rate of the fluid changes over time or a protocol in which saline is injected after the contrast medium is injected.

In the "protocol setting" mode, guidance may preferably be displayed to facilitate new registration or change of the injection protocol. By way of example, it is possible to use a setting screen which shows several items arranged in a line to be set as the injection protocol, and which allows the user to input a specific numeric value range and the like for each of the items to complete the registration or the change. The items may be arranged in a vertical direction or a horizontal direction.

The "environment setting" icon is provided for performing various types of setting. Examples of the setting include setting of a date and time, setting of sound volume, setting for cooperation (association) with another medical equipment, and setting of leak detection.

The "user editing" icon is provided for new setting and registration of a doctor, and for change of an already registered item.

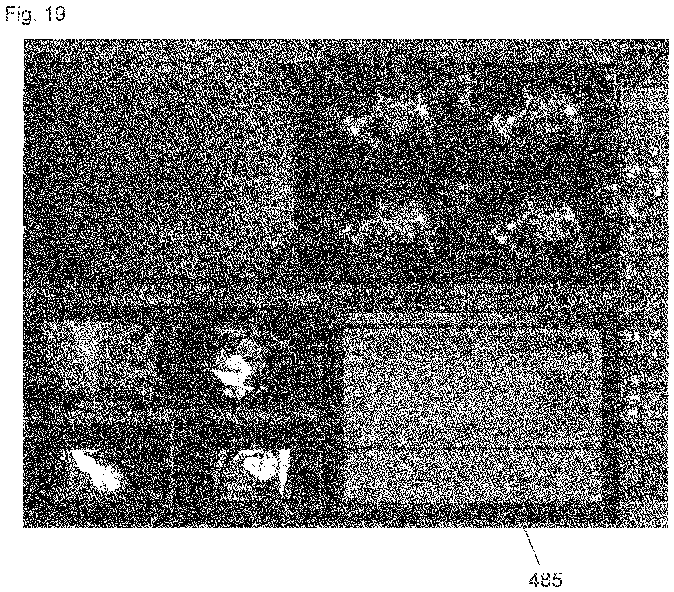

The icon of "injection results" is provided for displaying the results of the fluid injection. For example, it is possible to display an injection graph for a single fluid injection or to display a list of data of a plurality of fluid injections performed previously (by way of example, the date and time, the injection pattern, the injection rate, the injection amount, the injection pressure, and the product name of the contrast medium). Fig. 19 shows an example of cooperation of contrast medium injection apparatus 1 with an external medical system. In this example, graph 485 showing the results of fluid injection (by way of example, including information of what fluid was injected at what pressure for what period of time) may be externally output and displayed together with a diagnostic image shot by the imaging apparatus.

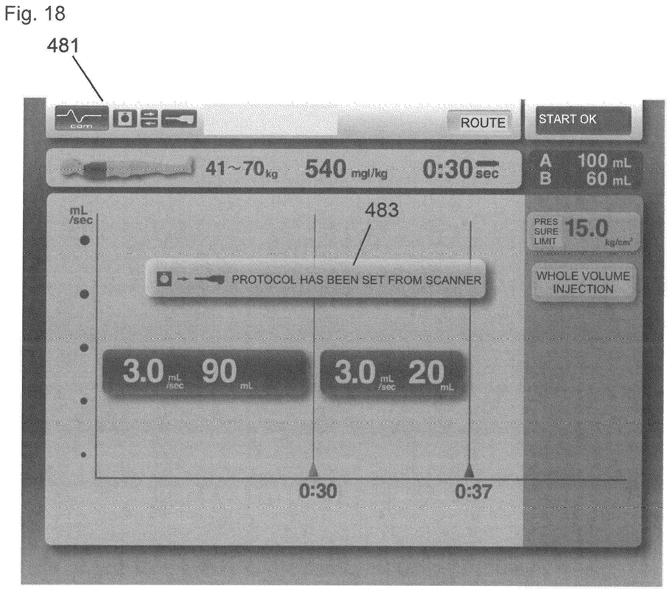

The following various screens may be displayed on console 150 of the present embodiment. Fig. 18 shows a state in which console 150 is connected to imaging apparatus 300. Image 481 is displayed on the upper left of the screen, indicating the connection to imaging apparatus 300. In this case, a predetermined injection condition protocol may be read from imaging apparatus (scanner) 300 and the protocol may be set as the injection condition. By way of example, a message, "protocol has been set from scanner," is displayed in message window 483 on the screen.

In the contrast medium injection apparatus of the present embodiment, a region screen registered as a default may be first displayed, and then the home screen may be displayed in response to pressing of a home button (a hard key on the console), for example. Instead of the pressing of the home button, a timer may be set to automatically display the home screen at a predetermined time.

The present section described above is as follows.
1. A contrast medium injection apparatus including an injector head for injecting a fluid from a syringe into a patient, and a console having a touch screen display and being capable of communicating with the injector head, the contrast medium injection apparatus configured to perform the following procedures;
   displaying a "home screen on" the display in which several icons are placed in matrix form, the home screen including icons for selecting an injection mode or condition setting methods; and
   when one of the icons is selected, displaying a screen for an injection mode relating to the selected icon or a screen for a condition setting method relating to the selected icon.
2. The contrast medium injection apparatus according to 1, wherein the icons further include an icon for "emergency mode" in which an injection will be performed in accordance with an injection condition of a preset injection rate and a preset injection time.
3. The contrast medium injection apparatus according to 2, wherein a guidance screen(s) is displayed on the injector head and/or the console in the emergency mode, for guiding a user in manipulation to be performed.
4. The contrast medium injection apparatus according to 2 or 3, wherein options for injection condition are configured to be displayed and one of them is selected, in the emergency mode.
5. The contrast medium injection apparatus according to 1 or 2, wherein the icons further include an icon for a "pediatric mode" for setting an injection condition for a child.
6. The contrast medium injection apparatus according to any one of 1 to 5, wherein the icons further include an icon for at least one mode selected from "weight input mode", "lean body weight mode", "body surface area mode", and "blood volume mode".
7. The contrast medium injection apparatus according to any one of 1 to 6, wherein the injector is configured display a doctor-selecting icon for selecting a doctor who uses the injector on the display.
8. The contrast medium injection apparatus according to 7, wherein, when one of the doctor-selecting icons is pressed, a condition-setting mode that has been determined will start.

### Section C: Other functions of injection apparatus

### (C-1: Self check)

The injection apparatus may be configured to perform the following operations.

The injection apparatus could automatically perform a series of operations called "self check" when a main power (main power of console 150, for example) is turned on. In the "self check," the piston driving mechanism 130 of injector head 110, some switches or sensors or the like will operate automatically to check whether normal operation can be performed or not. By way of example, to check the piston driving mechanism 130, a motor serving as the driving source thereof will actually rotate. In this case, the motor could require a few rotations for the checking, specifically, it is conceivable that a check is performed with smaller number of rotations than that used in fluid injections, such as a main injection for visualization or a pre-injection before the main injection.

Regarding switch and sensor or the like, the check is performed, for example by ensuring the conductive state of their parts or seeing whether or not its output value falls within a predetermined range. It may also be checked whether or not the connection between the devices is normal.

The results of the self check may preferably be displayed sequentially on the display as shown in Fig. 21. In the example of Fig. 21, items including "rate," "amount," "pressure," "stop," "switch," and "connection" are sequentially checked.

According to this configuration, since injector is used after normal operation of each function of the injector is ensured by the self check of the injector at the start-up thereof, it will be possible to prevent troubles due to a failure of the injector or an improper connection.

### (C-2: Guidance function in protocol setting)

While the description has been made in Fig. 13 of the display of the predetermined guidance image in response to the pressing of "i" button 453a as an example, such a guidance image may be displayed during the procedure of setting the injection protocol (injection conditions). For example, for an apparatus where several items need to be input to complete injection protocol, a guidance display for indicating next items to be input and the like could be displayed one by one. Such guidance images could be displayed automatically or displayed when user makes a predetermined input.

### (C-3: TBT mode)

It has been proposed to use "test injection method" or "bolus tracking method" to determine the scan timing in the imaging apparatus. Since the details of the bolus tracking method are described, for example in WO2011/136218 filed earlier by the present applicant, detailed description thereof is omitted. The outlines are as follows. In the bolus tracking method, an imaging apparatus monitors an increase in CT value of a certain Region Of Interest while the contrast medium is injected. If the CT value reaches a predetermined value, then the image scanning will be started after a given delay time. In the injection apparatus of the present embodiment, the bolus tracking mode may be started by touching the icon 414 on the screen in Fig. 12.

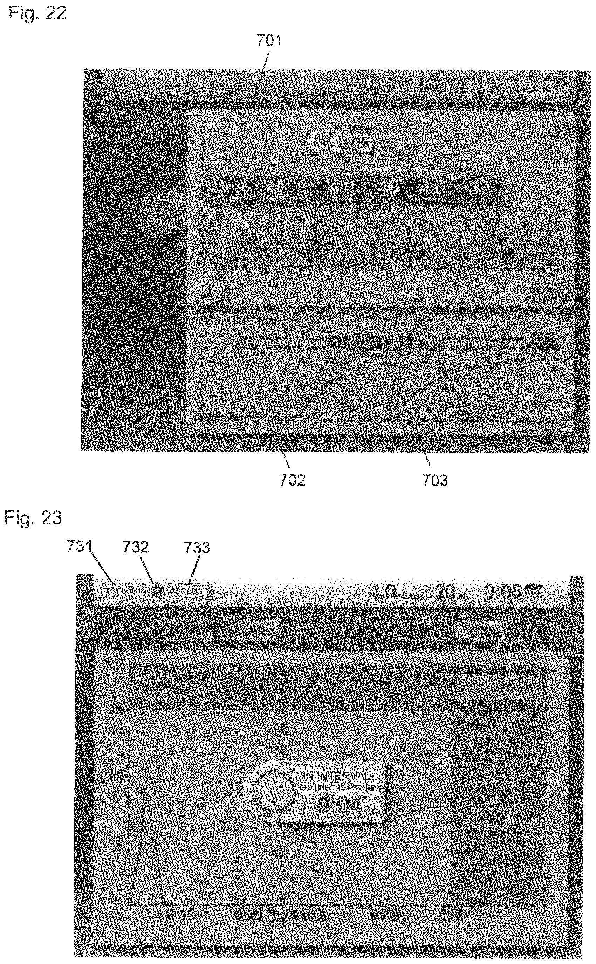

In the bolus tracking test, as described in WO2011/136218, a patient will hold his breath to stabilize the heart rate during a delay time after a first injection of contrast medium. In such a injection, for example as shown in Fig. 22, it is conceivable that a window is displayed on the display of the console, the window including an injection condition 701 relating to a saline and a contrast medium and a time line 702 of bolus tracking. In the window of time line 702, an area indicated by reference numeral 703 represents a time interval (delay time). Some character information may be displayed in the area 703, for example "delay," "breath held," and "stabilize heart rate." Each icon could include information time information (5 seconds for each in this case).

When an injection of the fluid is started, a current injection situation may be displayed for example as shown in Fig. 23. It is conceivable that indicators 731 to 733 are displayed in a status bar section in an upper portion of the screen to indicate currently performed process. The indicator 731 represents that "test bolus" injection is being performed, the indicator 732 represents that the operation is in the interval, and the indicator 733 represents that "bolus" injection is being performed. Fig. 23 shows indicator 732 highlighted to show that the operation is in the interval.

### (C-4: Data writing to external recording medium)

In the injection apparatus, after an injection is completed, information relating to the injection results or the like may be stored in an external storage medium. The storage medium may be a memory medium inserted into a predetermined slot (not shown) of the console. The written information is not limited particularly, and any information about the injection results may be written. The data may be written in CSV format so that the injection results are easily used in other personal computers or the like. The format of the data is not limited thereto, however, various formats can be used. The writing of the injection results is not limited to the storage medium such as a memory, the injection results may be sent to a predetermined system (for example, a hospital management system) via a network and may be saved on an internal storage apparatus there.

### (C-5: Display of information on injector head display)

. For example, when an injector head 110 includes a sub display (also referred to as a head display), it is conceivable that a graph relating to a pressure during injection, as shown in Fig. 9 is displayed on the head display. Specifically, the pressure graph may be displayed in real time on at least one of the main display and the head display, with synchronizing the console and the injector head. A pressure graph may be displayed on the head display during "route check", for example,. The "route review" is a mode for checking whether or not a route for fluid is established properly (i.e., whether an injection needle is properly inserted or not, or whether a kink has occurred in the tube or not). The apparatus in this embodiment, in which a user can check a pressure graph on the head display near the patient during "route review" mode, helps user detect an improper insertion of the injection needle and release the kink point.

The injection rate in the fluid injection for the route check could be automatically set to be the same as the injection rate in the main injection. Alternatively, the injection rate for the route check could be set independently of the injection rate in the main injection.

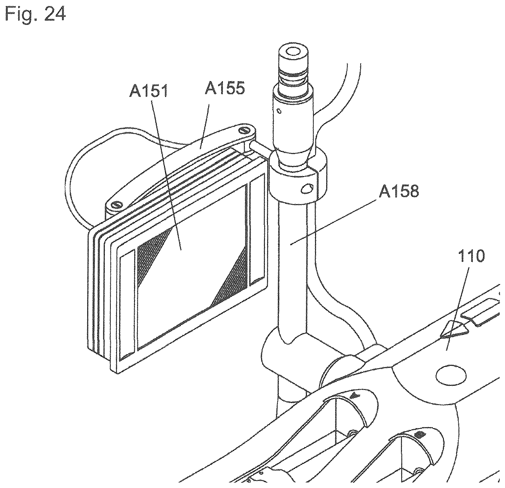

A head display as shown in Fig. 24 may be employed. In this configuration, an injector head 110 and a head display A151 are held on a head support structure A158. The head support structure A158 may be a conventionally known movable stand or a ceiling-mounted articulated support arm. The head display A151 is connected to the head support structure A158 through coupling mechanism A155. Although not limited particularly, the head display A151 may be located above the head with a space interval between the head display A151 and the injector head 110. The coupling mechanism A155 may hold the head display A151 for example such that the display becomes turnable about a vertical axis and/or a horizontal axis. According to such a configuration, since an orientation of the head display can be adjusted over a wide range, the operator visually recognizes the display more easily.

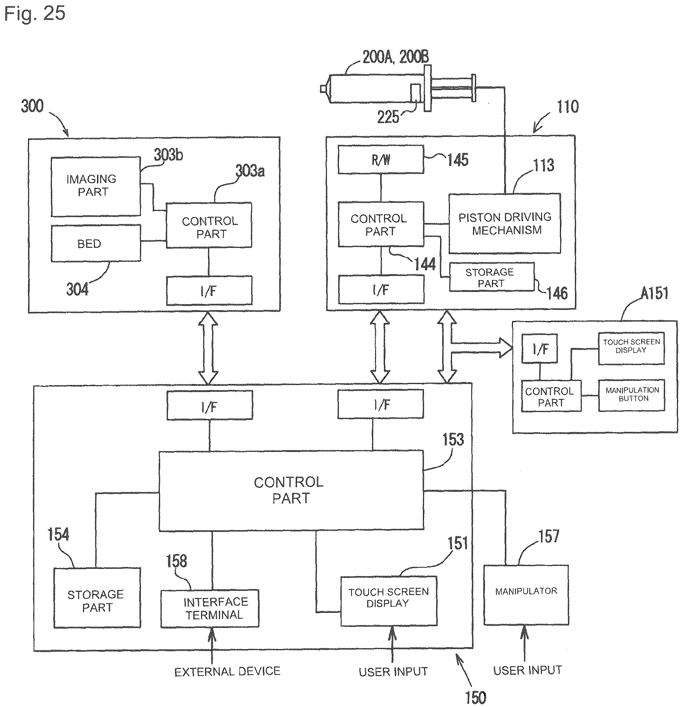

Head display A151 may be configured as shown in a block diagram of Fig. 25. Since the configuration in Fig. 25 is identical to that in Fig. 3A except for head display A151, redundant description is omitted. The head display A151 can communicate with the injector head 110 and the console 150 through an interface, and by way of example, may have a control part, a display of a touch panel type, and a manipulation button. The "manipulation button" may be a hard button provided for head display A151. However, head display A151 may not have such a manipulation button. In this manner, head display A151 may have various functions in the present invention.

The injector head may have no function of reading data from the identification tag on the syringe. In this case, for example, the console may have an input receiving part (not shown), which receives input from the operator, and information corresponding to at least part of the information on the identification tag may be configured to be input through the input receiving part. User may input a predetermined parameter by selecting from options displayed in a pull-down manner on the display.

### [DESCRIPTION OF REFERENCE NUMERALS]

- 110: INJECTOR HEAD
- 113: PISTON DRIVING MECHANISM
- 150: CONSOLE
- 151: DISPLAY
- 161: HUMAN BODY IMAGE
- 161a TO 161d: ICONS OF BODY SECTIONS
- 164: GROUP OF ICONS
- 164a to 164d: ICONS OF IMAGING REGIONS
- 169a to 169c: ICONS OF WEIGHT CLASSIFICATIONS
- 175: THUMBNAIL IMAGE
- 175a, 175b: CONDITION IMAGE
- 200A, 200B: SYRINGE
- 300: IMAGING APPARATUS
- 411 to 414: ICONS
- 415: ICON FOR DOCTOR SELECTION
- 416: ICON
- 450: WINDOW
- 453: GUIDANCE IMAGE

## Claims

1. An injection apparatus comprising a console having a touch screen display and a storage part (154), for injecting a contrast medium in a syringe into a patient and an injector head of a dual type, which performs injection of the contrast medium and injection of saline, the injection apparatus configured to perform the following procedures:
(a) displaying icons of imaging-regions on the display;
(b) when one icon of the imaging-regions is selected, reading an injection time from storage part (154) associated with the imaging-region;
(c) determining a contrast medium volume to be injected into a patient;
(d) determining an injection rate of the contrast medium based on the determined contrast medium volume and the injection time;
(e) displaying a thumbnail image (175) on the display, the thumbnail image (175) including a graph of an injection condition showing a relationship between the injection time and the injection rate; where
the graph of the thumbnail image (175) has a horizontal axis representing an elapsed time and a vertical axis representing the injection rate, and wherein a condition image (175a) is displayed at a vertical position corresponding to the injection rate and has a horizontal width corresponding to the injection time;
(f) when a certain icon on the display and/or a certain hard button on the injector head is pressed, starting a mode for an injection in accordance with the injection condition; **characterized in that** when the thumbnail image (175) is touched, the thumbnail image (175) is enlarged and another image (175') is shown, which is larger than the thumbnail image (175) and includes the first condition image (175a) of contrast medium, a second condition image (175b) of saline, and a syringe pressure limit image (173a'), said first condition image (175a) configured to be selectable so that a user can adjust the injection rate by moving said first condition image (175a) upward or downward;
(g) receiving input data, the data relating to at least an iodine concentration in the contrast medium;
(h) receiving an input relating to the patient's weight, and, in the procedure (c), the volume of the contrast medium is calculated on the basis of: the data of the input patient's weight, the data of the iodine concentration, and data of an iodine amount required per unit weight in association with the selected imaging-region.

2. The injection apparatus according to claim 1, wherein in the procedure (a), a human body image is displayed on the display prior to displaying the icons of the imaging-regions, and the icons of the imaging-region will be displayed in response to selecting one of the body-sections of said human body image.

3. The injection apparatus according to any one of claim 1 to 2, wherein in the processing of (h), icons for selecting classifications of patient's weight are displayed on the display and a classification is determined by selecting one of the icons.

4. The injection apparatus according to any one of claims 1 to 3, wherein a pressure graph of the contrast medium during injection is displayed on the display in real time, the graph including a horizontal axis representing an elapsed time and a vertical axis representing the injection pressure, wherein a region in the graph above a predetermined limit pressure value is displayed in a predetermined color or pattern, so as to make it easy to find of whether the injection pressure exceeds the predetermined limit pressure value.

## Patentansprüche

1. Eine Injektionsvorrichtung zum Injizieren eines Kontrastmittels in eine Spritze in einen Patienten, aufweisend eine Konsole mit einem berührungsempfindlichen Bildschirm und einen Speicherteil (154) und einen Injektorkopf eines dualen Typs, der eine Injektion des Kontrastmittels und von Kochsalzlösung durchführt, wobei die Injektionsvorrichtung zur Durchführung der folgenden Schritte ausgebildet ist:
(a) Anzeigen von Ikons von Abbildungsbereichen auf dem Bildschirm;
(b) wenn eines der Ikons von den Abbildungsbereichen ausgewählt wurde, Auslesen einer Injektionszeit aus dem mit dem Abbildungsbereich assoziierenden Speicherteil (154);
(c) Bestimmen eines einem Patienten zu injizierenden Kontrastmittelvolumens;
(d) Bestimmen einer Injektionsrate von dem Kontrastmittel auf der Grundlage des bestimmten Kontrastmittelvolumens und der Injektionszeit;
(e) Anzeigen eines Miniaturbildes (175) auf dem Bildschirm, wobei das Miniaturbild (175) einen Graphen einer Injektionsbedingung umfasst, die eine Beziehung zwischen der Injektionszeit und der Injektionsrate zeigt; wobei
der Graph des Miniaturbildes (175) eine horizontale Achse hat, die die verstrichene Zeit repräsentiert, und eine vertikale Achse hat, die die Injektionsrate repräsentiert, und wobei
ein Bedingungsbild (175a) in einer vertikalen Position angezeigt wird, die der Injektionsrate entspricht und eine horizontale Breite hat, die der Injektionszeit entspricht;
(f) wenn ein bestimmtes Ikon auf dem Bildschirm und/oder eine feste Taste an dem Injektorkopf gedrückt wird, Starten eines Modus für eine Injektion in Übereinstimmung mit der Injektionsbedingung;
**dadurch gekennzeichnet, dass**, wenn das Miniaturbild (175) berührt wird, das Miniaturbild (175) vergrößert wird und ein anderes Bild (175') gezeigt wird, welches größer als das Miniaturbild (175) ist und das erste Bedingungsbild (175a) des Kontrastmittels, ein zweites Bedingungsbild (157b) von Kochsalzlösung und ein Spritzendruckgrenzwertbild (173a') umfasst; wobei dieses erstes Bedingungsbild (175a) derart ausgebildet ist, dass dieses auswählbar ist, so dass ein Benutzer die Injektionsrate einstellen kann, indem dieses erste Bedingungsbild (175a) nach oben oder unten bewegt wird;
(g) Empfangen von Eingangsdaten, wobei die Daten sich auf wenigstens eine Jodkonzentration in dem Kontrastmittel beziehen;
(h) Empfangen einer Eingabe, die sich auf das Gewicht des Patienten bezieht, und in dem Schritt (c) das Volumen des Kontrastmittels auf der Grundlage der Daten des eingegebenen Gewichts des Patienten, den Daten der Jodkonzentration und den Daten einer Jodmenge, die für eine Gewichtseinheit benötigt wird, in Übereinstimmung mit dem ausgewählten Abbildungsbereich berechnet wird.

2. Die Injektionsvorrichtung nach Anspruch 1, wobei in dem Schritt (a) ein Bild des menschlichen Körpers auf dem Bildschirm angezeigt wird, bevor die Ikons von den Abbildungsbereichen angezeigt werden, und die Ikons von den Abbildungsbereichen in Abhängigkeit von der Auswahl eines der Körperbereiche des menschlichen Körperbildes angezeigt werden.

3. Die Injektionsvorrichtung nach einem irgendeinem der Ansprüche 1 oder 2, wobei in dem Schritt (h) Ikons zur Auswahl von Klassifikationen des Gewichts des Patienten auf dem Bildschirm angezeigt werden und eine Klassifikation durch die Auswahl eines der Ikons bestimmt wird.

4. Die Injektionsvorrichtung nach einem irgendeinem der Ansprüche 1 bis 3, wobei ein Druckgraph von dem Kontrastmittel während der Injektion auf dem Bildschirm in Echtzeit angezeigt wird, wobei der Graph eine horizontale Achse, die die verstrichene Zeit repräsentiert, und eine vertikale Achse, die den Injektionsdruck repräsentiert, umfasst, wobei ein Bereich in dem Graphen oberhalb eines vorbestimmten Grenzdruckwerts in einer vorbestimmten Farbe oder einem vorbestimmten Muster angezeigt wird, um die Feststellung, ob der Injektionsdruck den vorbestimmten Grenzdruckwert überschreitet, zu vereinfachen.

## Revendications

1. Appareil d'injection comprenant une console ayant un dispositif d'affichage à écran tactile et une partie de stockage (154), pour injecter un produit de contraste dans une seringue à un patient et une tête d'injection d'un type double, qui réalise une injection du produit de contraste et une injection de solution saline, l'appareil d'injection étant configuré pour réaliser les procédures suivantes :
(a) l'affichage d'icônes de régions d'imagerie sur le dispositif d'affichage ;
(b) lorsqu'une icône des régions d'imagerie est sélectionnée, la lecture d'un temps d'injection depuis la partie de stockage (154) associée à la région d'imagerie ;
(c) la détermination d'un volume du produit de contraste à injecter à un patient ;
(d) la détermination d'un débit d'injection du produit de contraste sur la base du volume déterminé du produit de contraste et du temps d'injection ;
(e) l'affichage d'une image de vignette (175) sur le dispositif d'affichage, l'image de vignette (175) incluant un graphique d'une condition d'injection montrant une relation entre le temps d'injection et le débit d'injection ; où le graphique de l'image de vignette a un axe horizontal représentant un temps écoulé et un axe vertical représentant le débit d'injection, et dans lequel une image de condition (175a) est affichée à une position verticale correspondant au débit d'injection et a une largeur horizontale correspondant au temps d'injection ;
(f) lorsqu'une certaine icône sur le dispositif d'affichage et/ou un certain bouton matériel sur la tête d'injection est actionné, le démarrage d'un mode pour une injection selon la condition d'injection ;
**caractérisé en ce que**, lorsque l'image de vignette (175) est touchée, l'image de vignette (175) est agrandie et une autre image (175') est montrée, qui est plus grande que l'image de vignette (175) et inclut la première image de condition (175a) de produit de contraste, une seconde image de condition (175b) de solution saline, et une image de limite de pression de seringue (173a'), ladite première image de condition (175a) configurée pour être sélectionnable de sorte qu'un utilisateur peut ajuster le débit d'injection en déplaçant ladite première image de condition (175a) vers le haut ou vers le bas ;
(g) la réception de données d'entrée, les données concernant au moins une concentration d'iode dans le produit de contraste ;
(h) la réception d'une entrée concernant le poids du patient, et, dans la procédure (c), le volume du produit de contraste est calculé sur la base de :
les données du poids entré du patient, les données de la concentration d'iode et des données d'une quantité d'iode requise par unité de poids en association avec la région d'imagerie sélectionnée.

2. Appareil d'injection selon la revendication 1, dans lequel, dans la procédure (a), une image de corps humain est affichée sur le dispositif d'affichage avant l'affichage des icônes des régions d'imagerie, et les icônes de la région d'imagerie seront affichées en réponse à la sélection d'une des sections de corps de ladite image de corps humain.

3. Appareil d'injection selon l'une quelconque des revendications 1 et 2, dans lequel, dans le traitement de (h), des icônes pour sélectionner des classifications de poids du patient sont affichées sur le dispositif d'affichage et une classification est déterminée par sélection d'une des icônes.

4. Appareil d'injection selon l'une quelconque des revendications 1 à 3, dans lequel un graphique de pression du produit de contraste durant une injection est affiché en temps réel sur le dispositif d'affichage, le graphique incluant un axe horizontal représentant un temps écoulé et un axe vertical représentant la pression d'injection, dans lequel une région dans le graphique au-dessus d'une valeur de pression limite prédéterminée est affichée selon une couleur ou un motif prédéterminé afin de faciliter le fait de constater si la pression d'injection excède la valeur de pression limite prédéterminée.
